# EUROPEAN PATENT SPECIFICATION

(11) **EP 3 538 163 B1**
(45) Date of publication and mention of the grant of the patent: **06.11.2024**
(21) Application number: 17793974.1
(22) Date of filing: 08.11.2017
(51) Int. Cl.: A61L 2/18, B61B 10/02, B65G 47/54

(54) **MACHINE FOR TREATING OBJECTS, IN PARTICULAR FOR WASHING, THERMAL DISINFECTION AND/OR STERILIZATION OF OBJECTS**
MASCHINE ZUR BEHANDLUNG VON OBJEKTEN, INSBESONDERE ZUM WASCHEN, ZUR THERMISCHEN DESINFEKTION UND/ODER ZUM STERILISIEREN VON OBJEKTEN
MACHINE DE TRAITEMENT D'OBJETS, EN PARTICULIER DE LAVAGE, DE DÉSINFECTION THERMIQUE ET/OU DE STÉRILISATION D'OBJETS

(30) Priority: 08.11.2016 IT 201600112539
(43) Date of publication of application: 18.09.2019
(73) Proprietor: Steelco S.p.A., 31039 Riese Pio X (IT)
(72) Inventor: CAPOVILLA, Ivone, 31037 Loria (IT)
(74) Representative: Petraz, Gilberto Luigi
(86) International application number: PCT/EP2017/078597
(87) International publication number: WO 2018/087144

(56) References cited:
- EP-A2- 1 787 730
- EP-A2- 1 787 731
- EP-A2- 1 980 474
- WO-A1-2015/145383
- WO-A1-2016/074066
- US-A- 3 595 373

## Description

### FIELD OF THE INVENTION

The present invention concerns a machine for treating objects, in particular a so-called "tunnel" treatment machine, in which successive steps of treating objects can be carried out in one or more treatment chambers in which corresponding treatment zones are disposed one after the other.

The term treatment can include "in their entirety" both operations to pre-treat the objects, such as pre-washing, with hot or cold water and/or with detergents or other chemical products, as well as washing operations proper, and also drying operations. The term "treatment" also includes thermal disinfection, sterilization, generally in autoclaves, and decontamination by means of particularly aggressive and dangerous decontamination substances, detergents and/or chemical agents such as for example peracetic acid.

By way of example, the objects that can be treated in the treatment machine in question can be instruments used in the medical field, in the laboratory, for analysis or research, instruments used in the pharmaceutical field, or medical instruments, surgical instruments or similar or comparable instruments, but does not exclude the application of the present invention to the treatment of objects in general.

### BACKGROUND OF THE INVENTION

Machines are known for treating objects, generally contained in one or more object-carrying containers such as racks or suchlike which are fed inside at least one suitable treatment chamber by feed means, for example a belt, where the desired treatment cycle is performed.

Normally, at least one door is provided, associated with an entrance aperture, which is mobile and driven by an opening and closing mechanism to assume a high or open position in which it allows the objects to be treated to enter into the treatment chamber, and a low or closed position, in which, in cooperation with fluidic sealing means, it seals the washing chamber from the outside.

In single-door treatment machines, the entrance and exit of the containers and the objects treated that are contained therein are carried out through the same door. In pass-through treatment machines, two doors are provided, that is, one door associated with an entrance aperture, and one door associated with an exit aperture, normally in the opposite position to the entrance aperture, respectively for the entrance and exit of containers and the treated objects contained therein.

As we said, one or more object-carrying containers can be inserted inside the treatment chamber of the machine. When several object-carrying containers are inserted, they are positioned sequentially one after the other in the chamber.

In the case of a "single-door" treatment machine, a roller is provided, on which to place the object-carrying container or containers and situated in correspondence with the door of the machine. From that door, then, the containers with the objects to be treated enter and the containers with the treated objects exit.

In the case of a "pass-through" treatment machine, generally two rollers are provided, positioned on diametrically opposite sides of the machine, of which one side has an entrance door for containers with objects to be treated and the other side has an exit door for the containers with treated objects.

A first disadvantage of known treatment machines is the need to carry out at least the action of loading the object-carrying container or containers in a manual way, that is, by substantially pushing the container or containers with the objects to be treated on a roller, until the latter have completely entered the treatment chamber of the machine.

Another disadvantage of known treatment machines, moreover, especially for pass-through machines, is the need to provide, in correspondence with the roller located on the exit side of the container or containers containing treated objects, another slider to collect the container or containers. The slider is generally positioned transversely with respect to the roller that supports the container or containers with treated objects, so it is obvious that the bulk required on the exit side of the machine is rather high, especially if a battery of "pass-through" machines is provided in parallel.

It should also be underlined that in certain machines for treating objects, for example thermal disinfection machines and even more so in sterilization machines, the operating conditions inside the object treatment chamber are rather difficult, for example because of very high temperatures, for example up to 140°C, but also because of the working pressures and/or the substances used for the treatment. It is therefore a good rule that these sterilization machines should have, inside the treatment chamber, no motorized or automatic moving means of the object-carrying containers and/or sensors to detect their position. This constraint, therefore, involves considerable complications at least in the development of technical solutions for moving object-carrying containers from and to the treatment chambers.

Document WO-A-2015/145383 describes an apparatus to introduce and extract containers to/from a sterilization machine which provides attachment elements provided at one end of each of the racks and able to allow, if necessary, the reciprocal attachment of the racks.

Therefore, the apparatus does not describe an apparatus for moving object-carrying containers which provides attachment or joining means or elements that are activated alternately and allow the insertion and extraction of one or more containers to/from the treatment chamber.

Therefore, even the above apparatus, although it is advantageous if several containers located in sequence are used, to avoid, for example, jamming of the object-carrying containers, can have the disadvantages described above.

Document EP-A-1787730 concerns an apparatus which is provided with a rotating device to rotate the loading plane of a washing machine, in order to automatically discharge any residual water.

This document too does not describe a machine for treating objects provided with an effective movement apparatus for the containers inside it, provided in particular with attachment elements suitable to function alternately.

Other movement apparatuses which suffer from these disadvantages are also described in US-A-3595373, which describes the use of magnetic means for positioning pallets on a conveyor, EP-A-1787731, which concerns another known washing machine, and WO-A-2016/074066, which describes a known industrial washing system.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to obtain a machine for treating objects, in particular washing, thermal disinfection and/or sterilization of objects, which can overcome at least one of the disadvantages of the state of the art.

In particular, one purpose of the present invention is therefore to obtain a machine for treating objects provided with means to move one or more object-carrying containers, which means, in both the steps of insertion and extraction to/from the machine, eliminate or at least substantially limit manual operations.

It is another purpose of the present invention to obtain a treatment machine which has advantageously reduced bulk and which requires limited spaces, compared with known machines, in zones adjacent to the loading and/or unloading door or doors of one or more object-carrying containers.

Another purpose of the present invention is to obtain a treatment machine, in particular a pass-through machine, which is provided with means to insert and extract one or more containers which allow it to load the one or more containers with the objects to be treated on one side of the machine and which allow to recover said one or more empty containers from the same side of the machine, once the treated objects have been unloaded on the opposite side of the machine, from which said one or more containers temporarily exit in order to pick up the treated objects.

Another purpose of the present invention is to obtain an effective and automatic apparatus to move one or more object-carrying containers that allows to insert and extract the one or more object-carrying containers to/from any machine for treating objects, which can be driven alternately for the insertion or extraction of said one or more containers.

Another purpose of the present invention is to perfect a fast, effective and safe method to move one or more object-carrying containers, both in the insertion step and in the extraction of said one or more containers from a machine for treating objects.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a machine for treating objects in particular for washing, thermal disinfection and /or sterilization of objects is according to appended claims.

The invention also concerns a method to move one or more object-carrying containers in a treatment machine, in particular a machine for washing, thermal disinfection and/or sterilization of objects, according to appended claims.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is partial three-dimensional view of a treatment machine for objects, for example a "pass-through" machine, provided with a movement apparatus to move one of more object-carrying containers;

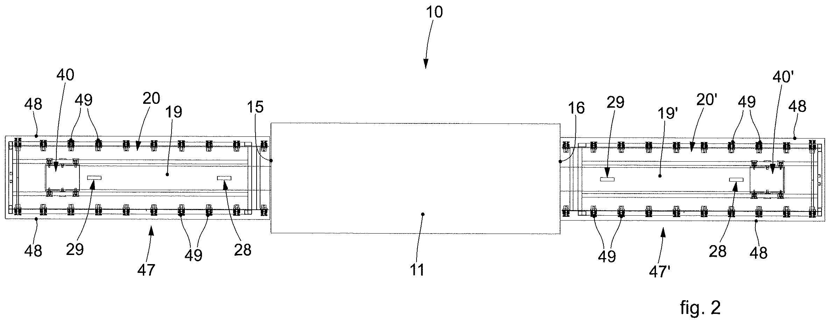

- fig. 2 is a plan view of the treatment machine in fig. 1;
- fig. 3 is a plan view, partly in section, of the movement apparatus of the object-carrying containers;
- fig. 4 is a lateral elevation view, partly in section, of the movement apparatus in an extraction configuration of the object-carrying container or containers from the machine;
- fig. 5 is a lateral elevation view, partly in section, of the movement apparatus in an insertion configuration of the object-carrying container or containers into the machine;
- figs. 6a-6d show some insertion and extraction steps of a pair of object-carrying containers into and from the treatment machine in fig. 1 and fig. 5.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

With reference to the attached drawings, a treatment machine 10 comprises at least one treatment chamber 11 in which one or more object-carrying containers, for example the object-carrying containers 12 and 13, can be positioned.

The object-carrying containers 12 and 13 can be in the form of racks or suchlike.

Typically, the object-carrying containers 12 and 13 are provided, substantially at the base, with a series of bars 14.

The bars 14 are suitably located at an adequate reciprocal distance, generally to facilitate the passage of liquids and/or fluids, and are directed in a substantially transverse direction with respect to a direction for inserting and extracting the object-carrying containers 12 and 13 into and out of the chamber 11. The bars 14 can also be provided in cross-configuration, although portions of the bars 14 are provided that are transverse as specified above.

Alternately, it would be possible to provide only one object-carrying container, or more than two object-carrying containers located in sequence.

The fact that two or more object-carrying containers 12 and 13 located in sequence are provided is useful, for example, if the user has problems of bulk for the operations of inserting and extracting the object-carrying containers from the treatment machine 10, therefore providing a single object-carrying container could cause movement problems.

The length of the object-carrying container or the object-carrying containers 12 and 13 located in sequence is normally such that substantially the whole length of the treatment chamber 11 is occupied.

Hereafter in the description, reference will be made, by way of non-restrictive example, to the example shown of two object-carrying containers 12 and 13 located in sequence, and a pass-through 10 treatment machine. As will be easily appreciated, however, the present invention can also refer to a single-door treatment machine and/or the use of a single object-carrying container or the use of more than two object-carrying containers.

The treatment chamber 11 will be provided inside with means, not shown in the drawings, useful for performing a specific treatment of the objects put in the object-carrying containers 12 and 13, for example washing, thermal disinfection, sterilization means or other.

The chamber 11 of the treatment machine 10, in this case by way of example a "pass-through" machine, comprises an aperture 15 for inserting the object-carrying containers 12, 13 with the objects to be treated and an aperture 16 for extracting the object-carrying containers 12, 13 with the objects treated.

The apertures 15 and 16 are positioned on opposite sides of the chamber 11 and are opened or closed by means of corresponding doors, not shown in the drawings, which can be driven manually or automatically.

The opening and closing doors 15 and 16 are normally sliding doors, for example shutter doors or suchlike, and are able to ensure adequate sealing once the chamber 11 has been closed to treat objects located in the object-carrying containers 12, 13.

Inside the treatment chamber 11, the object-carrying containers 12, 13 rest on a pair of supports 17, for example a pair of parallel tracks.

The supports 17 are suitably raised with respect to the base 18 of the treatment chamber 11 and between them and the base 18 a compartment 46 is made, to temporarily house at least part of a mobile support 19, 19' of a movement apparatus 20, 20' to move the object-carrying containers 12 and 13.

The movement apparatus 20, 20' is configured to assume a first active position in which it inserts the object-carrying containers 12, 13 inside the chamber 11, and at least a second active position in which it extracts the object-carrying containers 12, 13 from the chamber 11.

The first and second active positions of the apparatus 20, 20' are obtained by means of suitable joining means 28, 29 which can be selectively activated, alternately with respect to each other, in order to be associable on each occasion, depending on the operation of loading, unloading or transfer/return to be performed, to the object-carrying container or object-carrying containers 12, 13.

In possible implementations, there is a plurality of joining means 28, 29, that is, two or more, for each apparatus 20, 20'.

In possible implementations, the joining means 28, 29 of each apparatus 20, 20' include at least a first attachment element 28 and a second attachment element 29. In specific implementations, each apparatus 20, 20' comprises only two joining means 28, 29, that is, only a first attachment element 28 and a second attachment element 29.

In some embodiments, it is possible to provide that the first attachment element 28 is distanced from the second attachment element 29 at least by the length of an object-carrying container 12 or 13.

In the case shown by way of example, the first attachment element 28 is distanced from the second attachment element 29 for a segment equal to the length of two object-carrying containers 12, 13.

In some embodiments, which can be combined with all the embodiments and implementations described here, for each apparatus 20, 20' the first active position of the apparatus 20, 20' is obtained, as we will see, by activating a first attachment element 28 and by deactivating a second attachment element 29.

In some embodiments, which can be combined with all the embodiments and implementations described here, for each apparatus 20, 20' the second active position of the apparatus 20, 20' is obtained by activating the second attachment element 29 and deactivating the first attachment element 28.

In particular, the first active position includes an attachment position of one of said attachment elements 28, 29 to the object-carrying container 12, 13, and the second inactive position comprises a release position of the other of the attachment elements 29, 28 from the object-carrying container 12, 13.

It is understood that with the term attachment elements 28 and 29 we mean both mechanical joining means, for example attachment elements of the hook or anchor type, like those shown in the drawings, and also possibly magnetic joining means, able to be activated for the insertion and extraction of the object-carrying container, or a different kind of mechanical, electromechanical or magnetic or other means or elements.

In possible implementations, the attachment elements 28 and 29 can be activated in the sense that they are mobile to be selectively, and alternately, associable with the specific object-carrying container 12, 13.

Hereafter in the description, by way of non-restrictive example, reference will be made to the attachment elements 28 and 29 shown as selectively activated joining means.

The mobile support 19, 19' substantially comprises a box-like body that develops in the direction of insertion and extraction of the object-carrying containers 12 and 13 to/from the treatment chamber 11, and thus in a substantially longitudinal direction.

Inside the mobile support 19, 19' a shaft 21, 21' is housed, on which at least one pair of cams 22 and 23 are positioned.

The shaft 21, 21' can be rotated in one direction or the other by an actuator 34 associated with one end 35 of the shaft 21, 21' that emerges from the mobile support 19, 19'.

The actuator 34 is associated with the end 35 of the shaft 21, 21' by means of suitable motion transmission means 36, 36'.

The actuator 34, by lifting and lowering the corresponding rod 37, can allow a certain rotation of the shaft 21, 21' in one direction or the other. Such rotation can be, for example, about 90°.

The actuator 34, the shaft 21, 21' and the cams 22, 23 thus represent a drive unit of the attachment elements 28 and 29, configured to automatically change the active and inactive position, so that when one of the attachment elements 28, 29 is driven in the active position, the other attachment element 29, 28 automatically moves to the inactive position, and vice versa.

The drive unit 34, 21, 21', 22, 23, which essentially allows to convert or transform the rotational motion of the shaft 21, 21' into a lifting or lowering motion of the attachment elements 28, 29 could also be made in a different way to that shown, therefore it could provide actuation means, in particular mechanical motion conversion or transformation means, different from the cams 22 and 23 cooperating with the corresponding attachment elements 28 and 29.

The drive unit could therefore also be selected, for example, from a group comprising: screw actuators such as a screw jack, ball screw actuators and roller screw actuators, or a wheel and axle, for example a drum, gear, pulley or shaft, actuators such as a lifting cable, a winch, a rack and a pinion group, a chain transmission, a belt or strap transmission, rigid chain actuators and rigid belt or strap actuators, or others.

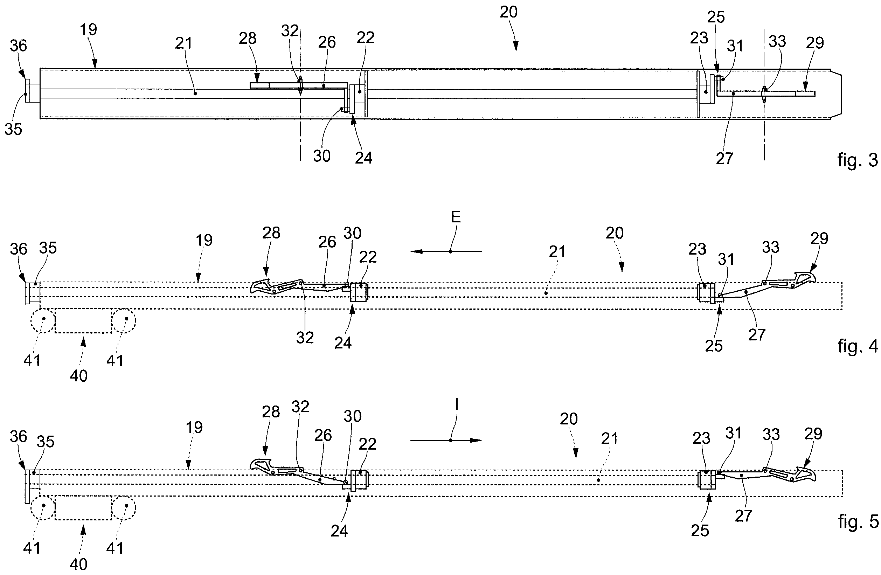

Therefore, returning to the description, as shown by way of non-restrictive example, each of the cams 22 and 23 of the shaft 21, 21' is connected, by means of a corresponding lever mechanism 24 and 25, to an end 26 and 27 of an attachment element 28 and 29, see in particular fig. 3, in which the box-like body of the mobile support 19 is shown without the upper part, so as to observe the inside.

The end 26 of the attachment element 28 is connected to the lever mechanism 24 by means of a rotation pin 30. The rotation pin 30 is raised and lowered along with the lever mechanism 24 and the end 26 of the attachment element 28.

The end 27 of the attachment element 29 is connected to the lever mechanism 25 by means of a rotation pin 31. The rotation pin 31 is raised and lowered along with the lever mechanism 25 and the end 27 of the attachment element 29.

Each of the attachment elements 28 and 29 is connected to a fixed rotation pin 32 and 33. The rotation pin 32, 33 is supported by the box-like body of the mobile support 19, 19'.

The attachment element 28 is able to allow an insertion movement in direction I of the object-carrying containers 12 and 13 inside the chamber 11, fig. 5, while the attachment element 29 is able to allow an extraction movement in direction E of the object-carrying containers 12 and 13 from the treatment chamber 11, fig. 4.

To this end, the attachment element 28 can have a concavity facing in direction I and the attachment element 29 can have a concavity facing in direction E.

The cams 22 and 23 and hence their corresponding lever mechanisms 24 and 25 are disposed in positions offset with respect to the shaft 21, 21', for example in diametrically opposite positions, see in particular fig. 3.

The attachment elements 28 and 29 protrude alternately from suitable slits 38 and 39 made on the surface of the mobile support 19, 19'.

The drive of the actuator 34, as will be seen below, automatically allows to lift and lower the attachment elements 28 and 29 alternately: therefore, by rotating the shaft 21, 21' in a first direction, following a first travel of the actuator 34, there will be a lifting of the attachment element 29 into an active position and a lowering of the attachment element 28 into an inactive position; on the contrary, by rotating the shaft 21, 21' in the other direction, following a second travel of the actuator 34, in the opposite direction to the first travel, there will be a lowering of the attachment element 29 into an inactive position and a lifting of the attachment element 28 into an active position.

As we said above, therefore, the rotational movement of the shaft 21, 21' is converted into an alternate lifting or lowering movement of the attachment elements 28, 29.

When one of the attachment elements 28, 29 is raised and hence in an active position, it is able to engage with one of the bars 14 of the object-carrying containers 12, 13, while the other attachment element 29, 28 is lowered and in the inactive position, thus substantially housed retracted inside the mobile support 19, 19' and thus in a non-interfering position with the object-carrying containers 12, 13.

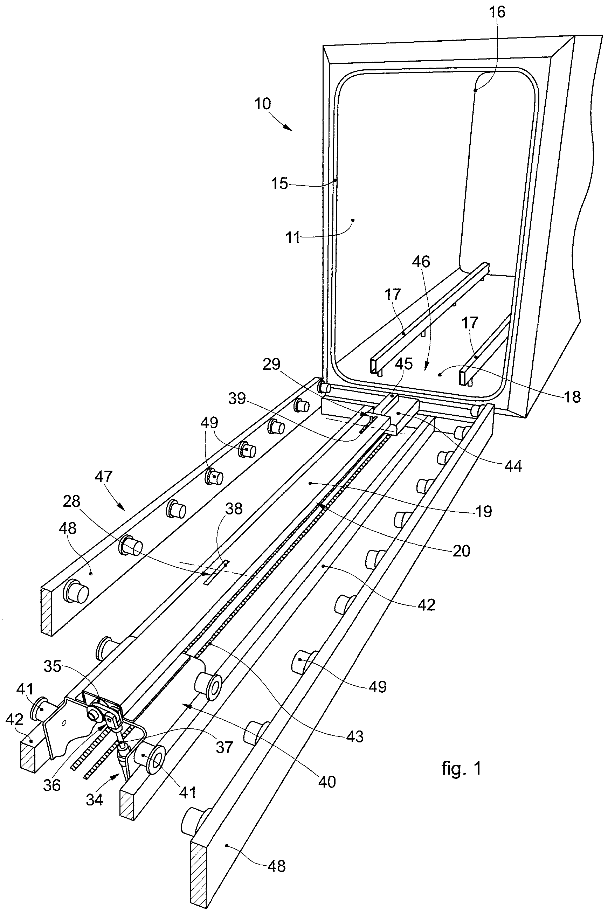

In fig. 1, by way of example, it has been assumed that the attachment element 29 is raised and in the active position, while the attachment element 28 is lowered and in the inactive position.

The mobile support 19, 19' of the movement apparatus 20, 20' is housed on a slider 40, 40'.

On each side the slider 40, 40' comprises rollers or wheels 41, resting on corresponding sliding guides 42.

The slider 40, 40' can be translated along the sliding guides 42, in one direction or the other, and by any suitable drive mean, for example an electric motor or other.

The slider 40, 40', in this example, is associated with a branch of a flexible motion transmission element 43, for example a chain, belt, or suchlike, wound at least around one pair of return wheels, one of which will be connected to said suitable drive mean.

The mobile support 19, 19', near the aperture 15, 16 of the treatment chamber 11, can rest on a support 44.

On the support 44, a ramp 45 can be made, for inserting the mobile support 19, 19' of the movement apparatus 20, 20' into the compartment 46 made between the supports 17.

The apparatus 20, 20' for moving the object-carrying containers 12, 13, can therefore be moved substantially bi-directionally along the guides 42, so that at least part of it, and in particular the mobile support 19, 19', is housed in the compartment 46 and in such a way that the object-carrying containers 12, 13 are correctly positioned resting on the supports 17.

The object-carrying containers 12, 13 outside the treatment chamber 11 rest on corresponding rollers 47, 47': a first roller 47, located in correspondence with the entrance aperture 15 of the object-carrying containers 12, 13 with the objects to be treated in the treatment room 11; and a second roller 47', located in correspondence with the exit aperture 16 of the object-carrying containers 12, 13, with the objects treated in the treatment chamber 11.

Each roller 47, 47' comprises at least one pair of guides 48 provided with rollers 49: the object-carrying containers 12, 13 are supported and can slide on said rollers 49.

The object-carrying containers 12, 13, for example racks, in which the objects to be treated are disposed, are positioned on the roller 47 which is located near the entrance aperture 15 of the treatment chamber 11. The door associated with the aperture 15 in this case will be open. On the contrary, it can be assumed that the door associated with the aperture 16 is closed.

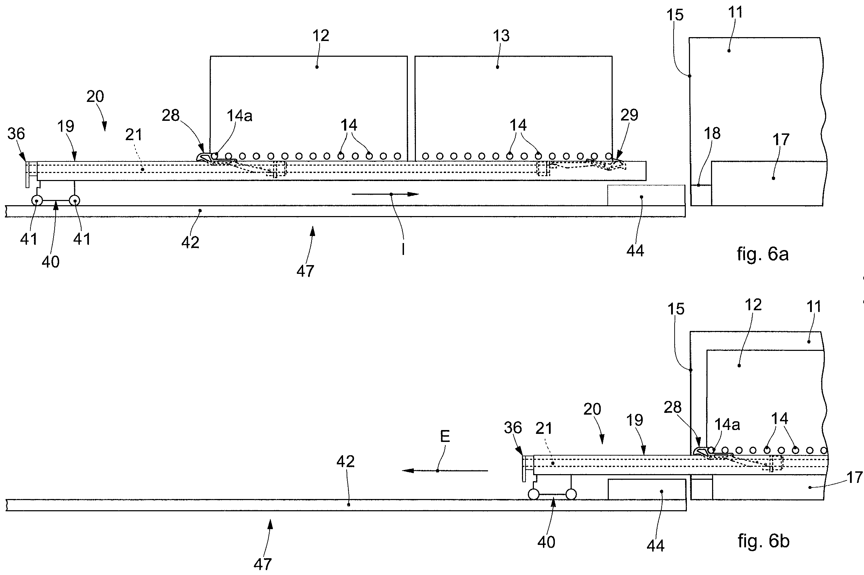

The object-carrying containers 12, 13 are positioned on the roller 47 in sequence and so that one of the transverse bars 14 of the first object-carrying container, that is, the object-carrying container 12, rests against the first attachment element 28, positioned in the raised position, see fig. 6a. The bar that cooperates with the attachment element 28 can be, for example, the first bar 14a. The attachment element 29, on the other hand, is in a lowered position and therefore does not interfere with the object-carrying containers 12, 13.

Lifting the attachment element 28 and lowering the attachment element 29 has been carried out, preferably prior to positioning the object-carrying containers 12, 13 on the roller 47, by means of the actuator 34.

In particular, to pass for example from the configuration shown in fig. 4 to that in fig. 5, hence to activate the attachment element 28 and deactivate the attachment element 29, the actuator 34 is driven, for example, so as to recall the rod 37 and to cause a certain lowering of the transmission elements 36; this lowering of the transmission elements 36 causes a rotation of the shaft 21, with a consequent rotation of the cam 22 by a certain angle and a lowering of the lever mechanism 24. The lowering of the lever mechanism 24 determines the rotation of the attachment element 28 around the fixed pin 32, so as to rise and become active, as in fig. 5. Also, the cam 23 associated with the attachment element 29 rotates by a certain angle, but being suitably offset with respect to the cam 22, the rotation of the shaft 21 will result in a lifting of the lever mechanism 25. The lifting of the lever mechanism 25 determines the rotation of the attachment element 29 around the fixed pin 33, so as to lower and become inactive, as in fig. 5.

The actuator 34, as we said, can cause the shaft 21 to rotate in the opposite direction to the one described above, so that the actuator 34, for example, by removing the rod 37, could again cause the lifting and the active configuration of the attachment element 29 and the consequent lowering and the inactive configuration of the attachment element 28, so as to pass from the configuration shown in fig. 5 to the configuration in fig. 4.

Let us therefore suppose that we are in the situation of fig. 6a, with the bar 14a of the object-carrying container 12 engaged with the attachment element 28 in the active position.

The slider 40 is driven in direction I so that it slides along the guides 42 and so that the mobile support 19 is at least partly inserted between the two supports 17, that is, in the compartment 46 of fig. 1. The attachment element 28 is substantially configured to thrust and move the first object-carrying container 12 inside the chamber 11 through the aperture 15.

The object-carrying container 12 slides along the roller 47 and pushes the object-carrying container 13, which also slides on the roller 47.

The slider 40 of the drive apparatus 20 is stopped when, substantially, the attachment element 28 is located in correspondence with the entrance aperture 15 of the chamber 11, or has just gone past it, see fig. 6b.

In this situation, both the object-carrying containers 12 and 13 are correctly positioned inside the chamber 11 and resting on the corresponding supports 17.

The mobile support 19 of the movement apparatus 20 can then be extracted from the chamber 11: the slider 40 is in this case translated in direction E, fig. 6b, so that it comes out completely from the chamber 11 and it is thus possible to close the door of the aperture 15.

In the step of extracting the mobile support 19 and hence the movement apparatus 20, the attachment element 28 is kept in the active position, as it does not interfere with the bars 14 and 14a of the object-carrying container 12. The other attachment element 29 is therefore kept in the inactive position.

Once the mobile support 19 of the movement apparatus 20 has been completely removed and the door of the aperture 15 has been closed, the treatment of the objects contained in the object-carrying containers 12 and 13 is performed inside the chamber 11. Of course, the door of the aperture 16 will also be closed.

At the end of the treatment of the objects inside the chamber 11, the door of the exit aperture 16 of the object-carrying containers 12, 13 is opened, with the objects treated.

The movement apparatus 20' is driven so that the slider 40' takes the mobile support 19' inside the chamber 11. In this step, the attachment element 29 of the apparatus 20' will be in the inactive position, therefore lowered, while the attachment element 28 of the apparatus 20' will be in the active position, therefore raised.

When the mobile support 19' has been properly inserted into the compartment made between the supports 17 of the chamber 11 through the aperture 16, the slider 40' is stopped and then the actuator associated with the transmission elements 36 of the apparatus 20' is driven. The actuator can be totally similar to the actuator 34 shown in fig. 1 and associated with the apparatus 20.

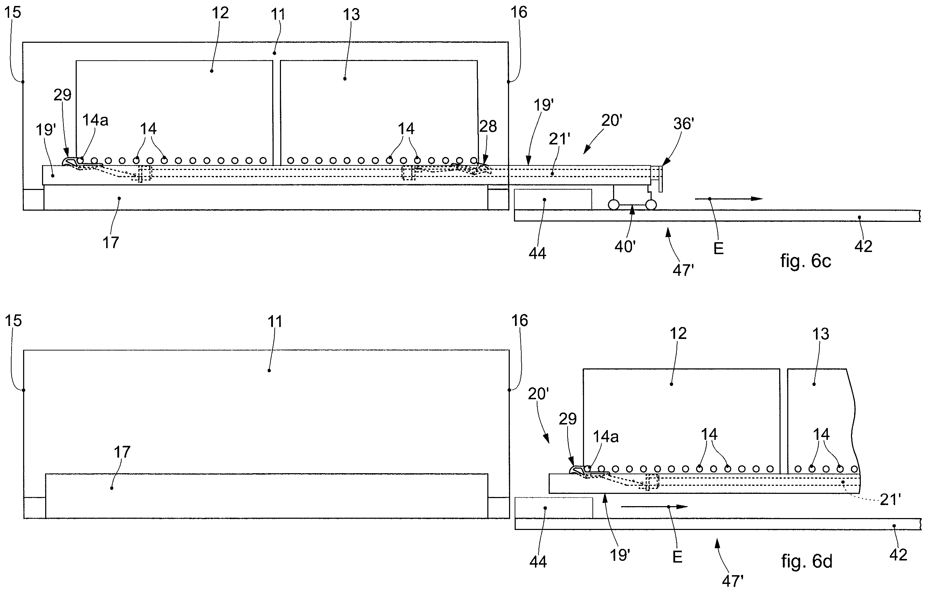

By means of this actuator, the shaft 21' disposed inside the mobile support 19', fig. 6c, is rotated so that the attachment element 29 assumes an active position and the attachment element 28 assumes an inactive position.

The attachment element 29 of the apparatus 20' thus cooperates with one of the transverse bars 14 of the object-carrying container 12, for example the bar 14a: by driving the slider 40' in direction E, the movement apparatus 20' extracts the object-carrying containers 12 and 13 from the chamber 11. This extraction takes place thanks to the attachment element 29 of the apparatus 20' which engages with the object-carrying container 12 which in turn thrusts the object-carrying container 13 out of the chamber 11.

When extraction is completed, see fig. 6d, the object-carrying containers 12 and 13 with the treated objects are both on the roller 47'.

Once the object-carrying containers 12 and 13 have been emptied of the treated objects, it is possible, thanks to the apparatus 20, 20', to return the object-carrying containers 12, 13, this time empty, to the roller 47, from where they started with the objects to be treated.

The treatment chamber 11 in this step can thus be used as a container-passing apparatus to return the object-carrying containers 12, 13 to the side from which they started.

This aspect can be extremely advantageous when, in the proximity of the exit roller 47' of the object-carrying containers 12, 13 there is a limited space and in any case not suitable to maneuver the object-carrying containers 12, 13.

To allow the return of the object-carrying containers 12, 13 to the roller 47 from which they started, as can easily be understood, it is necessary to operate in the opposite way to what has just been described.

In particular, assuming that the door of the aperture 16 is still open after the extraction shown in fig. 6d, it can be assumed, as we said, that the object-carrying containers have been emptied of the treated objects.

At this point, by activating the attachment element 28 of the apparatus 20' and deactivating the attachment element 29 of the apparatus 20', it is possible to push the empty object-carrying containers 12, 13 inside the chamber 11.

After the insertion of the empty object-carrying containers 12, 13 inside the chamber 11 is completed, the mobile support 19' of the movement apparatus 20' is extracted from the aperture 16.

It should be noted that, preferably, when the empty object-carrying containers 12, 13 have been inserted inside the chamber 11, it is best to first close the door associated with the aperture 16 and then to open the door associated with the aperture 15, so as to prevent any possible contamination of the atmosphere of the "clean" side of the treatment machine 10 by the "dirty" side of the treatment machine 10, or respectively, the side where the roller 47' facing toward the aperture 16 and the side where the roller 47 is positioned facing the aperture 15.

In general, therefore, when the door associated with the aperture 16 is open, the door associated with the aperture 15 should remain closed, or vice versa, in order to prevent the "clean" side of the treatment machine 10 from entering into communication in any way with the "dirty" side of the treatment machine.

Once the empty object-carrying containers 12 and 13 have been inserted into the chamber 11, the door associated with the aperture 16 is closed.

At this point, the movement apparatus 20 located on the roller 47 near the aperture 15 again intervenes, the door of which can be opened because the door of the aperture 16 has been closed.

The movement apparatus 20 has the attachment element 28 active and the attachment element 29 deactivated, so the mobile support 19 is inserted under the empty object-carrying containers 12, 13 so that the attachment element 29, in the inactive position, moves under one of the bars 14 of the empty object-carrying container 13.

At this point, thanks to the actuator 34 and as explained above, the attachment element 29 of the movement apparatus 20 is taken to the active position while the attachment element 28 is taken to the inactive position, passing substantially from the situation shown in fig. 5 to the situation in fig. 4.

Thanks to the subsequent drive of the slider 40 in direction E, it is possible to extract the two empty object-carrying containers 12, 13 and position them correctly on the roller 47.

Thanks to the movement apparatus 20, 20' with a double attachment element 28 and 29, it is therefore possible to introduce object-carrying containers 12, 13 with objects to be treated on one side of a pass-through treatment machine 10 and extract them, once emptied, from the same side from which they were introduced, for example the side of the aperture 15 with its corresponding roller 47. The treated objects will instead be suitably removed from the opposite side of the machine, in this example the side of the aperture 16 with corresponding roller 47'.

It is obvious that the present movement apparatus 20, 20' can advantageously be used also for "single-door" type machines, that is, machines by means of which the object-carrying containers are loaded with objects to be treated on the same side as the unloading of the object-carrying containers with treated objects.

Assuming that a "single-door" treatment machine is being used, provided for example only with the aperture 15, it is easily understood that the apparatus 20 would allow, in an automated manner, both the insertion and the extraction of one or more object-carrying containers 12, 13 into/from the treatment chamber 11.

In the insertion step of the object-carrying containers 12, 13 into the chamber 11, the attachment element 28 is activated and the attachment element 29 is deactivated, see fig. 5, so that the mobile support 19 introduces the object-carrying containers 12, 13 into the chamber 11. The mobile support 19 would then be extracted and the door associated with the aperture 15 would be closed for treatment.

Upon completion of the treatment, the door associated with the aperture 15 would be opened again and the mobile support 19 would be introduced again into the compartment 46 under the object-carrying containers 12, 13, with the attachment element 28 always in the active position and the attachment element 29 always in the inactive position.

When the insertion of the mobile support 19 is complete, and thanks to the actuator 34, the attachment element 29 would be taken to the active position and the attachment element 28 would be taken to the inactive position, fig. 4, so as to engage the object-carrying container 13 and take both the object-carrying containers 12, 13 outside by extracting the device 19 from the chamber 11.

As we have seen, the extraction of the mobile support 19 is performed by driving the slider 40 in direction E, while the insertion of the mobile support is performed by driving the slider in direction I.

As previously mentioned and as easily understood, the movement apparatus 20, 20' described could also be used in the case of a single object-carrying container of suitable length.

Furthermore, by varying the number and/or positioning of the attachment elements 28 and 29 on the removably connected mobile support 19, 19', it would be possible to provide object-carrying containers of different lengths or object-carrying containers which do not substantially occupy the entire extension of the treatment chamber 11.

Since it is provided with a substantially mechanical movement apparatus 20, 20' and is able to be inserted and disengaged from the chamber 11, the present treatment machine can also operate under difficult and complex operating conditions, for example those of a sterilization machine, where the working temperatures can be rather high, even up to 140°C, and therefore, where it is not advisable to use motorized means for moving the object-carrying containers inside the chamber.

It is also clear that, although the present invention has been described with reference to some specific examples, a person of skill in the art shall certainly be able to achieve many other equivalent forms of treatment machine, in particular for sterilization, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Machine for washing, thermal disinfection and/or sterilization of objects, comprising at least one treatment chamber (11) configured to house at least one object-carrying container (12, 13), the machine comprising at least one movement apparatus (20, 20') to move the object-carrying container (12, 13) provided with at least a first and a second attachment element (28, 29) which can be selectively activated, alternately one to the other, in order to be attached to the object-carrying container (12, 13) and configured to make said apparatus (20, 20') assume at least a first active position of insertion of the object-carrying container (12, 13) inside the chamber (11) and at least a second active position of extraction of the object-carrying container (12, 13) from the chamber (11), said attachment elements (28, 29) cooperating with a corresponding drive unit (34, 21, 21', 22, 23), configured to automatically alternate the active and inactive position, so that, when one of said attachment elements (28, 29) is driven in the active position, the other attachment element (29, 28) automatically moves into the inactive position, and vice versa, the machine being **characterized in that** said attachment elements (28, 29) cooperate with a shaft (21, 21') housed in the movement apparatus (20, 20') and provided with at least a first cam (22) configured to activate/deactivate said first attachment element (28) and with at least a second cam (23) configured to deactivate/activate said second attachment element (29), or vice versa, said shaft (21, 21') being made to rotate in one direction or the other by means of a corresponding actuator (34), wherein the apparatus further comprises at least a mobile support (19, 19') housing said shaft (21, 21') and comprising a box-like body that develops in the direction of insertion and extraction of the object-carrying containers (12, 13) to/from the treatment chamber (11), and wherein each of the attachment elements (28, 29) is connected to a fixed rotation pin (32, 33) supported by the box-like body of the mobile support (19, 19').

2. Machine as in claim 1, **characterized in that** if said chamber (11) houses a plurality of object-carrying containers (12, 13) located in sequence, said apparatus (20, 20'), by means of at least one of the attachment elements (28, 29), is configured to engage, in said first active position, with the first object-carrying container (12) of said plurality of object-carrying containers (12, 13) and is configured to engage, in said second active position, with the last object-carrying container (13) of said plurality of object-carrying containers (12, 13).

3. Machine as in claim 1, **characterized in that** said first and second attachment elements (28, 29) comprise at least a first active position of engagement with the object-carrying container (12, 13), in which at one of the attachment elements (28, 29) is raised, and at least a second inactive position of disengagement and non-interference with the object-carrying container (12, 13), in which the other attachment element (29, 28) is lowered.

4. Machine as in any claim hereinbefore, **characterized in that** it comprises a first access aperture (15) to the treatment chamber (11) located on a first side of the machine and a second access aperture (16) to the treatment chamber (11) located on a second side of the machine, a first movement apparatus (20) being positioned in proximity to said first access aperture (15) and a second movement apparatus (20') being positioned in proximity to said second access aperture (16).

5. Machine as in claim 4, **characterized in that** said apertures (15, 16) are positioned on opposite sides of the treatment chamber (11), said first movement apparatus (20), disposed in proximity to said first aperture (15), being configured to insert into the chamber (11) one or more object-carrying containers (12, 13) with objects to be treated, and to extract from the chamber (11) said one or more object-carrying containers (12, 13) which have been emptied, and said second movement apparatus (20'), disposed in proximity to said second aperture (16), being configured to extract from the chamber (11) one or more object-carrying containers (12, 13) with objects which have been treated, and to insert into said chamber (11) said one or more object-carrying containers (12, 13) which have been emptied.

6. Method to move one or more object-carrying containers in a machine for washing, thermal disinfection and/or sterilization of objects as in any of the claims from 1 to 5, **characterized in that** said method comprises: the automated insertion of one or more object-carrying containers (12, 13) with objects to be treated inside a treatment chamber (11) by means of said movement apparatus (20); the extraction of the movement apparatus (20) from the chamber (11) and the consequent treatment of the objects in the closed chamber (11); the opening of the chamber (11), when treatment is completed, and the new insertion of the movement apparatus (20) inside the chamber; and the automated extraction of said one or more object-carrying containers (12, 13) with the objects which have been treated.

7. Method as in claim 6, **characterized in that** said step of inserting one or more object-carrying containers (12, 13) occurs from a first side of the chamber (11), by means of a first movement apparatus (20), and the step of extracting said one or more object-carrying containers (12, 13) with objects that have been treated occurs from a second side of the chamber (11), by means of a second movement apparatus (20').

8. Method as in claim 7, **characterized in that** it comprises another step of inserting into the chamber (11) said one or more object-carrying containers (12, 13) which have been emptied from said second side of the chamber (11) by means of said second movement apparatus (20') and a step of extracting from the chamber (11) said one or more object-carrying containers (12, 13) which have been emptied from the first side of the chamber (11) by means of said first movement apparatus (20).

## Patentansprüche

1. Maschine zum Waschen, zur thermischen Desinfektion und/oder zum Sterilisieren von Objekten, umfassend zumindest eine Behandlungskammer (11), die dafür ausgelegt ist, zumindest einen objekttragenden Behälter (12, 13) zu beherbergen, wobei die Maschine zumindest eine Bewegungsvorrichtung (20, 20') zur Bewegung des objekttragenden Behälters (12, 13) umfasst, die mit zumindest einem ersten und einem zweiten Anbringungselement (28, 29) versehen ist, die, eines zu dem anderen abwechselnd, selektiv aktiviert werden können, um am objekttragenden Behälter (12, 13) angebracht zu werden, und dafür ausgelegt sind, zu veranlassen, dass die genannte Vorrichtung (20, 20') zumindest eine erste aktive Stellung des Einfügens des objekttragenden Behälters (12, 13) in die Kammer (11) und zumindest eine zweite aktive Stellung des Ausziehens des objekttragenden Behälters (12, 13) aus der Kammer (11) einnimmt, wobei die genannten Anbringungselemente (28, 29) mit einer entsprechenden Antriebseinheit (34, 21, 21', 22, 23) zusammenwirken, die dafür ausgelegt ist, die aktive und inaktive Stellung automatisch abzuwechseln, so dass, wenn eines der genannten Anbringungselemente (28, 29) in die aktive Stellung getrieben wird, das andere Anbringungselement (29, 28) sich automatisch zur inaktiven Stellung bewegt, und umgekehrt, wobei die Maschine **dadurch gekennzeichnet ist, dass** die genannten Anbringungselemente (28, 29) mit einer Welle (21, 21') zusammenwirken, die in der Bewegungsvorrichtung (20, 20') aufgenommen ist und mit zumindest einem ersten Nocken (22), der dafür ausgelegt ist, das genannte erste Anbringungselement (28) zu aktivieren/deaktivieren, und mit zumindest einem zweiten Nocken (23), der dafür ausgelegt ist, das genannte zweite Anbringungselement (29) zu aktivieren/deaktivieren, oder umgekehrt, versehen ist, wobei die genannte Welle (21, 21') in eine Richtung oder in die andere Richtung mittels eines entsprechenden Stellglieds (34) zum Drehen gebracht wird, worin die Vorrichtung ferner zumindest eine bewegliche Abstützung (19, 19') umfasst, die die genannte Welle (21, 21') aufnimmt und einen schachtelähnlichen Körper umfasst, der in der Richtung des Einfügens und des Ausziehens der objekttragenden Behälter (12, 13) in die/aus der Behandlungskammer (11) verläuft, und worin jedes von der Anbringungselemente (28, 29) mit einem festen Drehstift (32, 33) verbunden ist, der vom schachtelähnlichen Körper der beweglichen Abstützung (19, 19') abgestützt ist.

2. Maschine nach Anspruch 1, **dadurch gekennzeichnet, dass,** wenn die genannte Kammer (11) eine Vielzahl von objekttragenden Behältern (12, 13) beherbergt, die nacheinander angeordnet sind, die genannte Vorrichtung (20, 20'), mittels zumindest eines der Anbringungselemente (28, 29), dafür ausgelegt ist, in der genannten ersten aktiven Stellung, mit dem ersten objekttragenden Behälter (12) der genannten Vielzahl von objekttragenden Behältern (12, 13) einzugreifen, und dafür ausgelegt ist, in der genannten zweiten aktiven Stellung, mit dem letzten objekttragenden Behälter (13) der genannten Vielzahl von objekttragenden Behältern (12, 13) einzugreifen.

3. Maschine nach Anspruch 1, **dadurch gekennzeichnet, dass** die genannten ersten und zweiten Anbringungselemente (28, 29) zumindest eine erste aktive Stellung von Eingriff mit dem objekttragenden Behälter (12, 13), in der eines der Anbringungselemente (28, 29) angehoben ist, und zumindest eine zweite inaktive Stellung von Loslösung und Nicht-Eingriff mit dem objekttragenden Behälter (12, 13), in der das andere Anbringungselement (29, 28) abgesenkt ist, umfassen.

4. Maschine nach einem der vorhergehenden Ansprüche, **dadurch gekennzeichnet, dass** sie eine erste Zugangsöffnung (15) zur Behandlungskammer (11), die an einer ersten Seite der Maschine gelegen ist, und eine zweite Zugangsöffnung (16) zur Behandlungskammer (11), die an einer zweiten Seite der Maschine gelegen ist, umfasst, wobei eine erste Bewegungsvorrichtung (20) in der Nähe der genannten ersten Zugangsöffnung (15) positioniert ist und eine zweite Bewegungsvorrichtung (20') in der Nähe der genannten zweiten Zugangsöffnung (16) positioniert ist.

5. Maschine nach Anspruch 4, **dadurch gekennzeichnet, dass** die genannten Öffnungen (15, 16) an entgegengesetzten Seiten der Behandlungskammer (11) positioniert sind, wobei die genannte erste Bewegungsvorrichtung (20), die in der Nähe der genannten ersten Öffnung (15) angeordnet ist, dafür ausgelegt ist, einen oder mehrere objekttragende Behälter (12, 13) mit Objekten, die zu behandeln sind, in die Kammer (11) einzufügen, und den einen oder die mehreren objekttragenden Behälter (12, 13), die geleert wurden, aus der Kammer (11) auszuziehen, und die genannte zweite Bewegungsvorrichtung (20'), die in der Nähe der genannten zweiten Öffnung (16) angeordnet ist, dafür ausgelegt ist, einen oder mehrere objekttragende Behälter (12, 13) mit Objekten, die behandelt wurden, aus der Kammer (11) auszuziehen, und den einen oder die mehreren objekttragenden Behälter (12, 13), die geleert wurden, in die Kammer (11) einzufügen.

6. Verfahren zum Bewegen von einem oder mehreren objekttragenden Behältern in einer Maschine zum Waschen, zur thermischen Desinfektion und/oder zum Sterilisieren von Objekten nach einem der Ansprüche 1 bis 5, **dadurch gekennzeichnet, dass** das genannte Verfahren umfasst: das automatisierte Einfügen von einem oder mehreren objekttragenden Behältern (12, 13) mit Objekten, die zu behandeln sind, in eine Behandlungskammer (11) mittels der genannten Bewegungsvorrichtung (20); das Ausziehen der Bewegungsvorrichtung (20) aus der Kammer (11) und die folgende Behandlung der Objekte in der geschlossenen Kammer (11); das Öffnen der Kammer (11), wenn die Behandlung beendet ist, und das neue Einfügen der Bewegungsvorrichtung (20) in die Kammer; und das automatisierte Ausziehen des genannten einen oder der mehreren objekttragenden Behälter (12, 13) mit den Objekten, die behandelt wurden.

7. Verfahren nach Anspruch 6, **dadurch gekennzeichnet, dass** der genannte Schritt des Einfügens eines oder mehrerer objekttragender Behälter (12, 13) von einer ersten Seite der Kammer (11) her, mittels einer ersten Bewegungsvorrichtung (20), erfolgt und der Schritt des Ausziehens des genannten einen oder der mehreren objekttragenden Behälter (12, 13) mit Objekten, die behandelt wurden, von einer zweiten Seite der Kammer (11) her, mittels einer zweiten Bewegungsvorrichtung (20'), erfolgt.

8. Verfahren nach Anspruch 7, **dadurch gekennzeichnet, dass** es einen weiteren Schritt des Einfügens des einen oder der mehreren objekttragenden Behälter (12, 13), die von der genannten zweiten Seite der Kammer (11) her geleert wurden, mittels der genannten zweiten Bewegungsvorrichtung (20') in die Kammer (11) und einen Schritt des Ausziehens des einen oder der mehreren objekttragenden Behälter (12, 13), die von der ersten Seite der Kammer (11) her geleert wurden, mittels der genannten ersten Bewegungsvorrichtung (20) aus der Kammer (11) umfasst.

## Revendications

1. Machine de lavage, de désinfection thermique et/ou de stérilisation d'objets, comportant au moins une chambre de traitement (11) configurée pour recevoir au moins un récipient de transport d'objets (12, 13), la machine comportant au moins un appareil de déplacement (20, 20') pour déplacer le récipient de transport d'objets (12, 13) pourvu d'au moins un premier et un second élément d'attache (28, 29) qui peuvent être sélectivement activés, de manière alternée l'un par rapport à l'autre, afin d'être attachés au récipient de transport d'objets (12, 13) et configurés pour faire prendre audit appareil (20, 20') au moins une première position active d'insertion du récipient de transport d'objets (12, 13) à l'intérieur de la chambre (11), et au moins une seconde position active d'extraction du récipient de transport d'objets (12, 13) de la chambre (11), lesdits éléments d'attache (28, 29) coopérant avec une unité d'entraînement (34, 21, 21', 22, 23) correspondante, configurée pour faire automatiquement alterner la position active et la position inactive, de sorte que lorsque l'un desdits éléments d'attache (28, 29) est entraîné dans la position active, l'autre élément d'attache (29, 28) se déplace automatiquement dans la position inactive, et vice versa, la machine étant **caractérisée en ce que** lesdits éléments d'attache (28, 29) coopèrent avec un arbre (21, 21') reçu dans l'appareil de déplacement (20, 20') et pourvu d'au moins une première came (22) configurée pour activer/désactiver ledit premier élément attache (28) et d'au moins une seconde came (23) configurée pour désactiver/activer ledit second élément attache (29), ou vice versa, ledit arbre (21, 21') étant amené à tourner dans un sens ou l'autre au moyen d'un actionneur (34) correspondant, dans laquelle l'appareil comporte en outre au moins un support mobile (19, 19') recevant ledit arbre (21, 21') et comportant un corps analogue à un boîtier qui se développe dans la direction d'insertion et d'extraction des récipients de transport d'objets (12, 13) vers/depuis la chambre de traitement (11), et dans laquelle chacun des éléments d'attache (28, 29) est relié à un axe de rotation fixe (32, 33) supporté par le corps analogue à un boîtier du support mobile (19, 19').

2. Machine selon la revendication 1, **caractérisée en ce que** si ladite chambre (11) reçoit une pluralité de récipients de transport d'objets (12, 13) situés en séquence, ledit appareil (20, 20'), au moyen d'au moins un des éléments d'attache (28, 29), est configuré pour venir en prise, dans ladite première position active, avec le premier récipient de transport d'objets (12) de ladite pluralité de récipients de transport d'objets (12, 13) et est configuré pour venir en prise, dans ladite seconde position active, avec le dernier récipient de transport d'objets (13) de ladite pluralité de récipients de transport d'objets (12, 13).

3. Machine selon la revendication 1, **caractérisée en ce que** lesdits premier et second éléments d'attache (28, 29) comportent au moins une première position active de prise avec le récipient de transport d'objets (12, 13), dans laquelle au moins un des éléments d'attache (28, 29) est levé, et au moins une seconde position inactive de libération et de non-interférence avec le récipient de transport d'objets (12, 13), dans laquelle l'autre élément d'attache (29, 28) est abaissé.

4. Machine selon l'une quelconque des revendications précédentes, **caractérisée en ce qu'**elle comporte une première ouverture d'accès (15) à la chambre de traitement (11) située sur un premier côté de la machine et une seconde ouverture d'accès (16) à la chambre de traitement (11) située sur un second côté de la machine, un premier appareil de déplacement (20) étant positionné à proximité de ladite première ouverture d'accès (15) et un second appareil de déplacement (20') étant positionné à proximité de ladite seconde ouverture d'accès (16).

5. Machine selon la revendication 4, **caractérisée en ce que** lesdites ouvertures (15, 16) sont positionnées sur des côtés opposés de la chambre de traitement (11), ledit premier appareil de déplacement (20), disposé à proximité de ladite première ouverture (15), étant configuré pour insérer dans la chambre (11) un ou plusieurs récipients de transport d'objets (12, 13) avec des objets à traiter, et pour extraire de la chambre (11) ledit ou lesdits récipients de transport d'objets (12, 13) qui ont été vidés, et ledit second appareil de déplacement (20'), disposé à proximité de ladite seconde ouverture (16), étant configuré pour extraire de la chambre (11) un ou plusieurs récipients de transport d'objets (12, 13) avec des objets qui ont été traités, et pour insérer dans ladite chambre (11) ledit ou lesdits récipients de transport d'objets (12, 13) qui ont été vidés.

6. Procédé pour déplacer un ou plusieurs récipients de transport d'objets dans une machine de lavage, de désinfection thermique et/ou de stérilisation d'objets selon l'une quelconque des revendications 1 à 5, **caractérisé en ce que** ledit procédé comporte l'insertion automatisée d'un ou plusieurs récipients de transport d'objets (12, 13) avec des objets à traiter à l'intérieur d'une chambre de traitement (11) au moyen dudit appareil de déplacement (20), l'extraction de l'appareil de déplacement (20) de la chambre (11) et le traitement conséquent des objets dans la chambre (11) fermée, l'ouverture de la chambre (11), lorsque le traitement est terminé, et la nouvelle insertion de l'appareil de déplacement (20) à l'intérieur de la chambre, et l'extraction automatisée dudit ou desdits récipients de transport d'objets (12, 13) avec les objets qui ont été traités.

7. Procédé selon la revendication 6, **caractérisé en ce que** ladite étape d'insertion d'un ou plusieurs récipients de transport d'objets (12, 13) a lieu depuis un premier côté de la chambre (11), au moyen d'un premier appareil de déplacements (20), et l'étape d'extraction dudit ou desdits récipients de transport d'objets (12, 13) avec des objets qui ont été traités a lieu depuis un second côté de la chambre (11), au moyen d'un second appareil de déplacement (20').

8. Procédé selon la revendication 7, **caractérisé en ce qu'**il comporte une autre étape consistant à insérer dans la chambre (11) ledit ou lesdits récipients de transport d'objets (12, 13) qui ont été vidés à partir dudit second côté de la chambre (11) au moyen dudit second appareil de déplacement (20') et une étape consistant à extraire de la chambre (11) ledit ou lesdits récipients de transport d'objets (12, 13) qui ont été vidés à partir du premier côté de la chambre (11) au moyen dudit premier appareil de mouvement (20).
